# EUROPEAN PATENT SPECIFICATION

(11) **EP 3 451 964 B1**
(45) Date of publication and mention of the grant of the patent: **30.06.2021**
(21) Application number: 17792992.4
(22) Date of filing: 13.04.2017
(51) Int. Cl.: A61B 18/18

(54) **SYSTEMS FACILITATING APPLICATION OF AN APPROPRIATE THERMAL DOSAGE IN MICROWAVE ABLATION PROCEDURES**
SYSTEME ZUR ERLEICHTERUNG DER ANWENDUNG EINER GEEIGNETEN THERMISCHEN DOSIERUNG BEI MIKROWELLENABLATIONSVERFAHREN
SYSTÈMES FACILITANT L'APPLICATION D'UNE DOSE THERMIQUE APPROPRIÉE DANS DES PROCÉDURES D'ABLATION PAR MICRO-ONDES

(30) Priority: 03.05.2016 US 201615144989
(43) Date of publication of application: 13.03.2019
(73) Proprietor: Covidien LP, Mansfield, MA 02048 (US)
(72) Inventor: BRANNAN, Joseph A., Lyons, CO 80540 (US)
(74) Representative: Maschio & Soames IP Ltd
(86) International application number: PCT/US2017/027411
(87) International publication number: WO 2017/192255

(56) References cited:
- EP-A2- 2 666 425
- WO-A1-2013/138262
- US-A1- 2006 184 163
- US-A1- 2009 196 480
- US-A1- 2009 198 309
- US-A1- 2011 208 180
- US-A1- 2012 095 461
- US-A1- 2014 378 967

## Description

### BACKGROUND

### 1. Technical Field

The present disclosure relates to systems and methods for planning and performing ablation treatment procedures and, more particularly, to systems and methods facilitating application of an appropriate thermal dosage to tissue during microwave ablation procedures.

### 2. Discussion of Related Art

Treatment of certain diseases requires the destruction of malignant tissue growths, e.g., tumors. Electromagnetic radiation can be used to heat and destroy tumor cells. Treatment may involve inserting ablation probes into or adjacent to tissues where cancerous tumors have been identified. Once the probes are positioned, electromagnetic energy is passed through the probes into surrounding tissue to treat, e.g., heat, ablate and/or coagulate tissue.

The volume of tissue to be treated often varies, as well the ability of that tissue (or adjacent tissue) to absorb heat. For effective treatment, heating of tissue should occur within certain temperature ranges. If the temperature is too high, adjacent healthy tissue may be damaged. If the temperature is too low, the treatment to the cancerous tissue may not be effective. As a result, it is common to use sensors in conjunction with an ablation apparatus to monitor tissue temperature. In existing systems, if the temperature reaches a certain threshold that may be injurious to the patient, the ablation apparatus will automatically shut down.

However, temperature monitoring in this manner does not consider how energy is absorbed into the body over time. For example, a patient may suffer irreversible tissue damage to healthy tissue in an ablation apparatus set below the temperature threshold, if the patient is exposed to that temperature for a prolonged period. User error may also result as the duration and temperature of the treatment is typically entered manually.

US 20091966480 discloses a thermal ablation system which is operable to perform thermal ablation using an x-ray system to measure temperature changes throughout a volume of interest in a patient. Image data sets captured by the x-ray system during a thermal ablation procedure provide temperature change information for the volume being subjected to the thermal ablation. Intermediate image data sets captured during the thermal ablation procedure may be fed into a system controller, which may modify or update a thermal ablation plan to achieve volume coagulation necrosis targets. The thermal ablation may be delivered by a variety of ablation modes including radiofrequency ablation, microwave therapy, high intensity focused ultrasound, laser ablation, and other interstitial heat delivery methods.

US 2006184163 discloses a system and method for modeling the death of tissue cells that are thermally treated using thermal treatment devices is disclosed. A cell-death model accurately predicts, in real-time, which voxels of cells are dead or are about to die as the thermal treatment is applied to these cells. The effects of thermal treatment are monitored by a thermal measurement device which feeds thermal information to the cell-death model. The cell-death model accounts for the temperature of each voxel of tissue cells with respect to a temperature threshold value and the duration over which the thermal treatment is applied. When the thermal measurement device is an imaging device, the results of the thermal treatment may be displayed to the user in real-time. As a result, a user of the thermal treatment device can determine, in real-time, which target voxels of cells have been killed and which still need to be killed. The user can also more easily avoid inadvertently killing healthy tissue that it is not intended to kill. The cell-death model may be implemented in software on the thermal measurement device, on the thermal treatment device, or on a separate processing device which interfaces to and communicates with at least one of the thermal measurement device and the thermal treatment device.

### SUMMARY

Provided in accordance with aspects of the present disclosure is a microwave ablation system including a microwave ablation probe configured to deliver energy to a target volume of tissue during an ablation procedure, at least one temperature sensor configured to determine a temperature of the target volume of tissue at a plurality of points in time during the ablation procedure, and a computing device operably coupled to the microwave ablation probe and the at least one temperature sensor. The computing device includes a processor and a memory storing instructions, which when executed by the processor, cause the computing device to load a temperature accumulation profile corresponding to the target volume of tissue, and dynamically control at least one setting of the microwave ablation probe in accordance with the temperature of the target volume of tissue at each of the points in time such that the temperature of the target volume of tissue follows the temperature accumulation profile during the ablation procedure.

In an aspect of the present disclosure, the temperature accumulation profile loaded is selected from a plurality of different temperature accumulation profiles corresponding to various different tissue characteristics.

In another aspect of the present disclosure, the at least one temperature sensor includes a plurality of temperature sensors.

In yet another aspect of the present disclosure, at least one of the plurality of temperature sensors is disposed on the microwave ablation probe.

In still another aspect of the present disclosure, at least one of the plurality of temperature sensors is a remote temperature probe.

In still yet another aspect of the present disclosure, the at least one setting of the microwave ablation probe includes at least one of, energy output from the microwave ablation probe, cooling of the microwave ablation probe, or cycling the microwave ablation probe on and off.

In another aspect of the present disclosure, the computing device includes a timer configured to correlate the temperature of the target volume of tissue at each of the points in time to an elapsed time of the ablation procedure.

In yet another aspect of the present disclosure, the computing device dynamically controls the at least one setting of the microwave ablation probe based upon the Arrhenius equation.

In still another aspect of the present disclosure, an ultrasound imager is configured to generate real-time ultrasound images.

Provided in accordance with another aspect of the present disclosure is a microwave ablation method including, selecting a temperature accumulation profile corresponding to a target volume of tissue to be ablated, inserting a microwave ablation probe into the target volume of tissue, performing an ablation procedure by activating the microwave ablation probe to deliver energy to the target volume of tissue, determining a temperature of the target volume of tissue at a plurality of points in time during the ablation procedure, and dynamically controlling, using a computing device, at least one setting of the microwave ablation probe in accordance with the temperature of the target volume of tissue at each of the points in time such that the temperature of the target volume of tissue follows the temperature accumulation profile during the ablation procedure.

In an aspect of the present disclosure, the temperature accumulation profile is selected from a plurality of different temperature accumulation profiles corresponding to various different tissue characteristics.

In another aspect of the present disclosure, the at least one setting of the microwave ablation probe includes at least one of, energy output from the microwave ablation probe, cooling of the microwave ablation probe, or cycling the microwave ablation probe on and off.

In yet another aspect of the present disclosure, the computing device dynamically controls the at least one setting of the microwave ablation probe based upon the Arrhenius equation.

In still yet another aspect of the present disclosure, real-time ultrasound images are generated to visualize the microwave ablation probe during the ablation procedure.

### BRIEF DESCRIPTION OF THE DRAWINGS

Objects and features of the present disclosure will become apparent to those of ordinary skill in the art when descriptions thereof are read with reference to the accompanying drawings, of which:
Fig. 1 is a schematic diagram of a microwave ablation planning and procedure system in accordance with the present disclosure;
Fig. 2 is a schematic diagram of a computing device which forms part of the microwave ablation planning and procedure system of Fig. 1 in accordance the present disclosure;
Fig. 3 is flow chart illustrating an example method of a procedure phase of a microwave ablation treatment in accordance with the present disclosure;
Fig. 4 is a flow chart illustrating an example of a treatment plan selection method for a microwave ablation planning and procedure system in accordance the present disclosure;
Fig. 5 is a graph illustrating temperature accumulation profiles for a microwave ablation planning and procedure system in accordance with the present disclosure;
Fig. 6 is a flowchart illustrating an example of a safety protocol method for a microwave ablation planning and procedure system in accordance with the present disclosure;
Fig. 7 is an illustration of a user interface presenting a view during the procedure phase of the microwave ablation treatment in accordance with the present disclosure; and
Fig. 8 is an illustration of a user interface presenting a view during an ablation step of the procedure phase of the microwave ablation treatment in accordance with the present disclosure.

### DETAILED DESCRIPTION OF THE EMBODIMENTS

It has been found that temperature accumulation profiles are useful in planning for and/or providing proper thermal dosage in a microwave ablation treatment procedure. The temperature accumulation profiles are also useful in providing adequate safety measures to help prevent damage to healthy tissue during ablation of target tissue. These and other aspects and features of the present disclosure are detailed herein below.

Microwave ablation treatment can generally be divided into two phases: (1) a planning phase, and (2) a procedure phase. Exemplary planning and procedure phases of microwave ablation treatment are described in U.S. Patent Application No. 14/821,912 entitled TREATMENT PROCEDURE PLANNING SYSTEM AND METHOD, filed on August 10, 2015 by Bharadwaj et al., U.S. Patent Application No. 14/821,950 entitled TREATMENT PROCEDURE PLANNING SYSTEM AND METHOD, filed on August 10, 2015 by Bharadwaj et al., International Patent Application No. PCT/US15/44659 entitled TREATMENT PROCEDURE PLANNING SYSTEM AND METHOD, filed on August 11, 2015 by Bharadwaj et al., and Provisional Patent Application No. 62/154,929 entitled MICROWAVE ABLATION PLANNING AND PROCEDURE SYSTEMS, filed on April 30, 2015 by Girotto. A microwave ablation planning and procedure system may be a unitary system configured to perform both the planning phase and the procedure phase, or the system may include separate devices and/or programs for the various phases. An example of the latter may be a system wherein a first computing device with one or more specialized programs is used during the planning phase, and a second computing device with one or more specialized programs imports data from the first computing device to be used during the procedure phase.

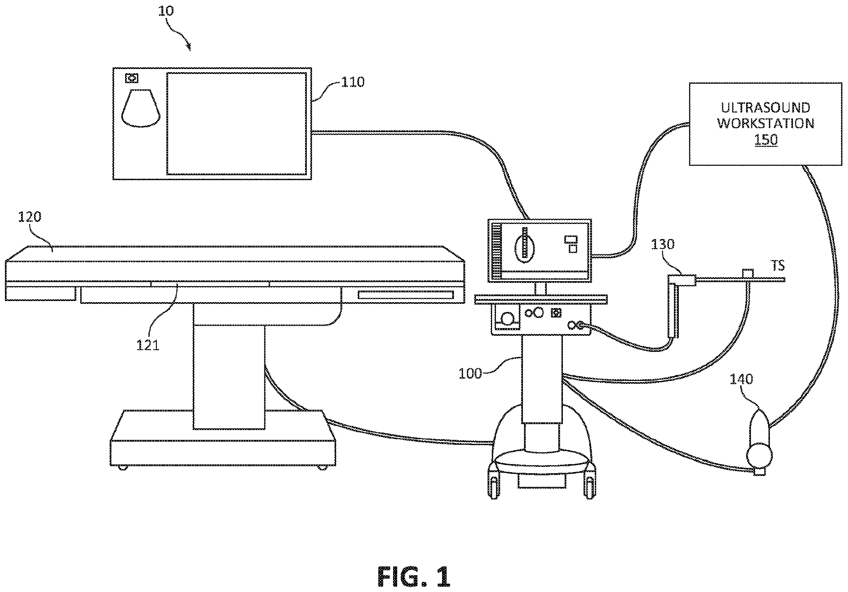

Referring now to FIG. 1, a treatment system 10 of the present disclosure includes a computing device 100, a display 110, a table 120, an ablation probe 130, and an ultrasound sensor 140. Computing device 100 may be, for example, a laptop computer, desktop computer, tablet computer, or other similar device. Computing device 100 may be configured to control an electrosurgical generator, a peristaltic pump, a power supply, and/or any other accessories and peripheral devices relating to, or forming part of, treatment system 10. Display 110 is configured to output instructions, images, and/or messages relating to the performance of the microwave ablation procedure. Table 120 may be, for example, an operating table or other table suitable for use during a surgical procedure, which includes an electromagnetic (EM) field generator 121. EM field generator 121 is used to generate an EM field during the microwave ablation procedure and forms part of an EM tracking system, which is used to track the positions of surgical instruments within the body of a patient. An example of such an EM tracking system is the AURORA™ system sold by Northern Digital Inc. of Waterloo, Ontario, Canada EM field generator 121 may include various components, such as a specially designed pad to be placed under, or integrated into, table 120.

Ablation probe 130 is a surgical instrument having a microwave ablation antenna, which is used to ablate tissue. While the present disclosure describes the use of treatment system 10 in a surgical environment, it is also envisioned that some or all of the components of treatment system 10 may be used in alternative settings, for example, an imaging laboratory and/or an office setting. It is also contemplated that the present disclosed be utilized with any other suitable treatment system.

In addition to the EM tracking system, the surgical instruments, e.g., ablation probe 130, may also be visualized by using ultrasound imaging work station 150. Ultrasound sensor 140, such as an ultrasound wand, may be used to image the patient's body during the microwave ablation procedure to visualize the location of ablation probe 130 inside the patient's body. Ultrasound sensor 140 may have an EM tracking sensor embedded within or attached to the ultrasound wand, for example, a clip-on sensor or a sticker sensor. Ultrasound sensor 140 may be positioned in relation to ablation probe 130 such that ablation probe 130 is at an angle to the ultrasound image plane, thereby enabling the clinician to visualize the spatial relationship of ablation probe 130 with the ultrasound image plane and with objects being imaged. Further, the EM tracking system may also track the location of ultrasound sensor 140. In some embodiments, one or more ultrasound sensors 140 may be placed inside the body of the patient. EM tracking system may then track the location of such ultrasound sensors 140 and ablation probe 130 inside the body of the patient.

Various other surgical instruments or surgical tools, such as other electrosurgical devices, surgical staples, etc., may also be used during the performance of a microwave ablation treatment procedure. Ablation probe 130 is used to ablate a lesion or tumor (hereinafter referred to as a "target") by using electromagnetic radiation or microwave energy to heat tissue in order to denature or kill cancerous cells. The construction and use of a system including such an ablation probe 130 is U.S. Patent Application No. 14/828,682 entitled MICROWAVE ABLATION SYSTEM, filed on August 18, 2015 by Dickhans, International Application No. PCT/US15/46729 entitled MICROWAVE ABLATION SYSTEM, filed on August 25, 2015 by Dickhans, U.S. Patent Application No. 13/836,203 entitled MICROWAVE ABLATION CATHETER AND METHOD OF UTILIZING THE SAME, filed on March 15, 2013 by Ladtkow et al., and U.S. Patent Application No. 13/834,581 entitled MICROWAVE ENERGY-DELIVERY DEVICE AND SYSTEM, filed on March 15, 2013 by Brannan et al.

The location of ablation probe 130 within the body of the patient may be tracked during the surgical procedure. An example method of tracking the location of ablation probe 130 is by using the EM tracking system, which tracks the location of ablation probe 130 by tracking sensors attached to or incorporated in ablation probe 130. Various types of sensors may be used, such as a printed sensor, the construction and use of which is described in U.S. Patent Application No. 14/919,950 entitled MEDICAL INSTRUMENT WITH SENSOR FOR USE IN A SYSTEM AND METHOD FOR ELECTROMAGNETIC NAVIGATION, filed on October 22, 2015 by Greenburg et al., and International Application No. PCT/US15/58320 entitled MEDICAL INSTRUMENT WITH SENSOR FOR USE IN A SYSTEM AND METHOD FOR ELECTROMAGNETIC NAVIGATION, tiled on October 30, 2015 by Greenburg et al. Prior to starting the procedure, the clinician is able to verify the accuracy of the tracking system.

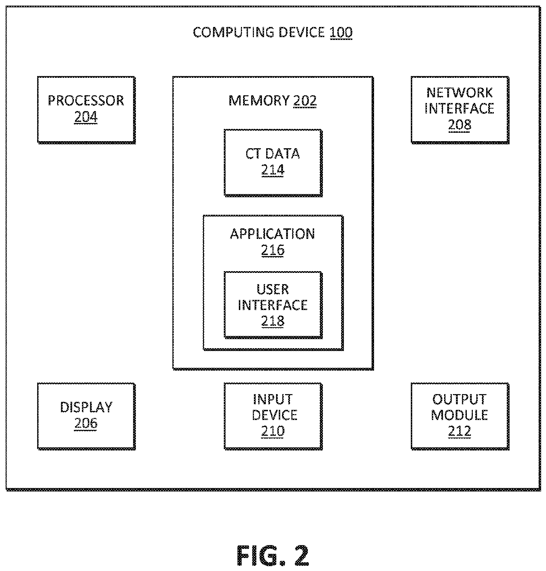

Turning now to FIG. 2, a system diagram of computing device 100 is depicted. Computing device 100 may include memory 202, processor 204, display 206, network interface 208, input device 210, and/or output module 212. Memory 202 includes any non-transitory computer-readable storage media for storing data and/or software that is executable by processor 204 and which controls the operation of computing device 100. Processor 204 may be a general purpose processor, a specialized graphics processing unit (GPU) configured to perform specific graphics processing tasks while freeing up the general purpose processor to perform other tasks, and/or any number or combination of such processors. Display 206 may be touch sensitive and/or voice activated, enabling display 206 to serve as both an input and output device. Alternatively, a keyboard (not shown), mouse (not shown), or other data input devices may be employed.

Network interface 208 may be configured to connect to a network such as a local area network (LAN) consisting of a wired network and/or a wireless network, a wide area network (WAN), a wireless mobile network, a Bluetooth network, and/or the internet. For example, computing device 100 may receive computed tomographic (CT) image data of a patient from a server, for example, a hospital server, internet server, or other similar servers, for use during surgical ablation planning. Patient CT image data may also be provided to computing device 100 via memory 202. Computing device 100 may receive updates to its software, for example, application 216, via network interface 208. Computing device 100 may also display notifications on display 206 that a software update is available. Input device 210 may be any device by means of which a user may interact with computing device 100, such as, for example, a mouse, keyboard, foot pedal, touch screen, and/or voice interface. Output module 212 may include any connectivity port or bus, such as, for example, parallel ports, serial ports, universal serial busses (USB), or any other similar connectivity port known to those skilled in the art.

Application 216 may be one or more software programs stored in memory 202 and executed by processor 204 of computing device 100. As will be described in more detail below, during the planning phase, application 216 guides a user through a series of steps to identify a target, size the target, size a treatment zone, proximity to other tissue structures, type of treatment, duration of treatment, and/or access routes to the target for later use during the procedure phase.

Application 216 may be installed directly on computing device 100, or may be installed on another computer, for example, a central server, and opened on computing device 100 via network interface 208. Application 216 may run natively on computing device 100, as a web-based application, or any other format known to those skilled in the art. In some embodiments, application 216 will be a single software program having all of the features and functionality described in the present disclosure. In other embodiments, application 216 may be two or more distinct software programs providing various parts of these features and functionality. For example, application 216 may include one software program for use during the planning phase, and a second software program for use during the procedure phase of the microwave ablation treatment. In such instances, the various software programs forming part of application 216 may be enabled to communicate with each other and/or import and export various settings and parameters relating to the microwave ablation treatment and/or the patient to share information. For example, a treatment plan and any of its components generated by one software program during the planning phase may be stored and exported to be used by a second software program during the procedure phase.

Application 216 communicates with a user interface 218, which generates a user interface for presenting visual interactive features to a user, for example, on display 206 and for receiving user input, for example, via a user input device. For example, user interface 218 may generate a graphical user interface (GUI) and output the GUI to display 206 for viewing by a user.

Referring also to FIG. 1, computing device 100 is linked to display 110, thus enabling computing device 100 to control the output on display 110 along with the output on display 206. Computing device 100 may control display 110 to display output which is the same as or similar to the output displayed on display 206. For example, the output on display 206 may be mirrored on display 100. Alternatively, computing device 100 may control display 110 to display different output from that displayed on display 206. For example, display 110 may be controlled to display guidance images and information during the microwave ablation procedure, while display 206 is controlled to display other output, such as configuration or status information.

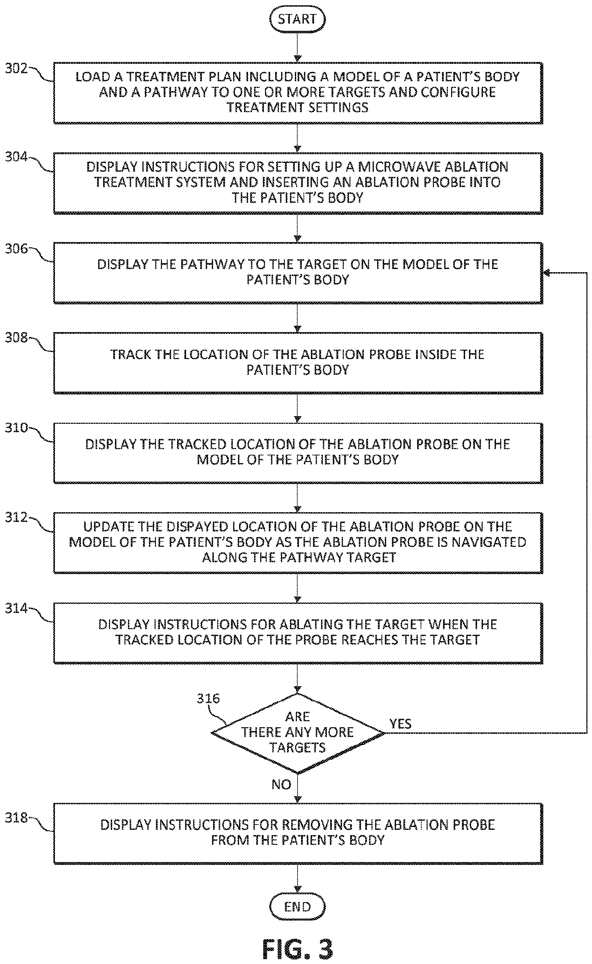

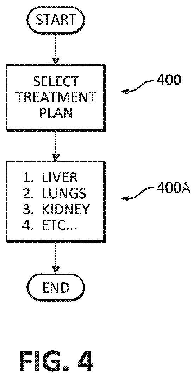

Turning now to FIG. 3, in conjunction with FIGS. 1 and 2, an example method for performing a microwave ablation procedure in accordance with the present disclosure is detailed. At step 302, a user may use computing device 100 to load a treatment plan 400 (FIG. 4) into application 216. The treatment plan 400 (FIG. 4, described in more detail below) may include various treatment plans for an ablation procedure, a model of a patient's body, and/or a pathway to one or more targets. Then, at step 304, application 216, via user interface 218, displays instructions for setting up and configuring the microwave ablation system. The instructions may be visual and/or audible, and may provide feedback for proper versus improper system configuration. Thereafter, at step 306, application 216 displays the model of the patient's body with the pathway to the target as was generated in the planning phase. While ablation probe 130 is navigated, application 216, at step 308, tracks the location of ablation probe 130 inside the patient's body, and, at step 310, displays the tracked location of ablation probe 130 on the model of the patient's body. Application 216, at step 312, iteratively updates the displayed location of ablation probe 130 on the model of the patient's body as ablation probe 130 is navigated along the pathway to the target. When application 216 or the clinician detects that ablation probe 130 has reached the target, application 216, at step 314, displays instructions for ablating the target according to the settings previously set for ablating the tumor.

Thereafter, at step 316, application 216 determines if there are any more targets in the treatment plan that have yet to be treated based on the planned procedure. If the determination is yes, the process returns to step 306 where the displayed pathway is updated to reflect the pathway to the next target. If the determination is no, application 216, at step 318, displays instructions for removing ablation probe 130 from the patient's body. During the ablation procedure, data relating to power and time settings as well as temperature data of ablation probe 130 for each ablation is continually stored.

With reference to FIG. 4, selection of a treatment plan 400 of step 302 is provided. Treatment plan 400 generally includes selection of a treatment best suited for a given ablation procedure based on the target's tissue type, the target's proximity to other tissue structures, the size of the target, and/or other characteristics of the target, patient, etc. Treatment plan 400 may include measuring and/or varying the thermal dosage to one or more targets during ablation in accordance with a temperature accumulation profile 400A. In an embodiment, treatment plan 400 may be a software program(s) or subprograms(s) loaded onto and/or integrated into application 216 of computing device 100. Treatment plan 400 may include menus and/or sub-menus such that a user may select an appropriate treatment plan 400 for a given ablation procedure. More specifically, the selection of treatment plan 400 may include selecting a temperature accumulation profile 400A to plan and prepare for an ablation procedure. For example, if the target is located in a patient's liver, then a user would select the temperature accumulation profile 400A for the liver. If the target was located in the patient's kidney, then a user would select a temperature accumulation profile 400A for the kidney, and so on and so forth. However, the temperature accumulation profiles 400A are not limited to categorization based solely on the location; rather, temperature accumulation profiles 400A may be categorized or sub-categorized according to other characteristics of the target tissue, ablation zone size, proximity of the target to other tissue structures, behavior of the target and adjacent tissue structures when subjected to energy over time, characteristics of the patient, etc. For example, if the target is located in a patient's liver, after selecting liver, a user may then be presented with a list of temperature accumulation profiles 400A, all of which are applicable to the ablation of liver tissue, but which are further categorized based upon other characteristics.

Data used to determine the various temperature accumulation profiles 400A may be gathered through empirical testing and/or predictive modeling, may be updated and/or refined based on feedback provided by treatment system 10 (FIG. 1) and/or the user, and may be stored in one or more data look-up tables in memory 202 of computing device 100, or in any other suitable fashion. Each temperature accumulation profile 400A provides a change in tissue temperature as a function of time that results in effective ablation of the target tissue (to which the temperature accumulation profile 400A corresponds) without damaging adjacent healthy tissue.

The Arrhenius equation, for example, may be used in determining the temperature accumulation profile 400A based upon observed data. The Arrhenius equation, more specifically, establishes a first-order exponential relationship between tissue exposure time, tissue temperature, and tissue injury based upon experimental cell survivability studies. Tissue damage can be described by the Arrhenius equation as Ω=A ∫*e*^{-E}ₐ^{/(RT)} *dt*, where Ω is the tissue damage during time of treatment t, A is the Arrhenius constant, Eₐ is the energy activation of the cells, R is the universal gas constant, and T is the temperature of the tissue. The tissue injury integral increases as the time of exposure increases. While certain tissues may have some degree of inhomogeneity, Arrhenius parameters have been determined for several body tissues commonly targeted in ablation procedures, such as, for example, the liver, lungs, and kidneys. It should be appreciated that temperature accumulation profiles 400A may be adapted to any type of tissue or set of characteristics of tissue, whether homogeneous or inhomogeneous, using any suitable algorithm.

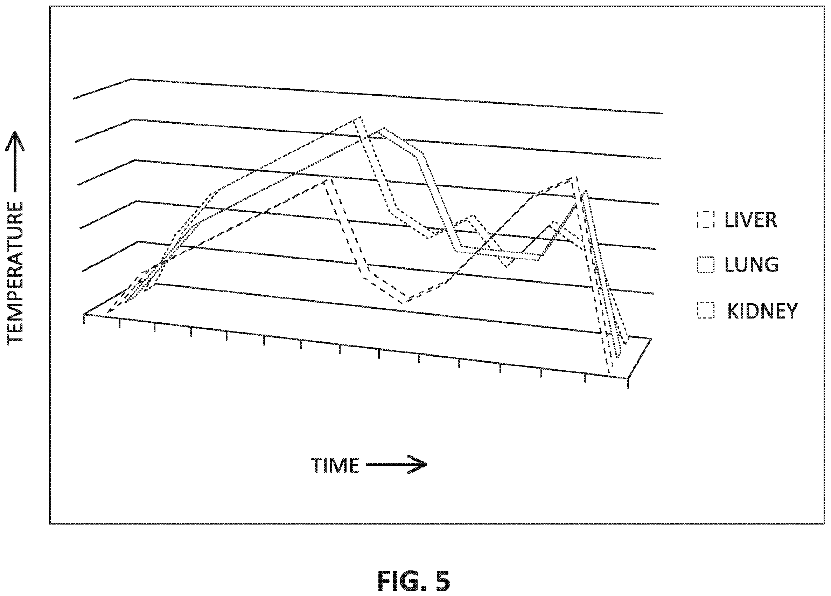

Referring also to FIG. 5, example temperature accumulation profiles 400A are depicted in graph format. The temperature accumulation profiles 400A in FIG. 5 are shown for purposes of understanding and are not representative or limiting. As can be seen by these temperature accumulation profiles 400A, a particular profile may provide different rates of temperature change, both positive and negative rates of change, and/or periods of constant temperature during the tissue ablation process in order to achieve effective ablation of the target tissue (to which the temperature accumulation profile 400A corresponds) without damaging adjacent healthy tissue.

In order to implement one of the temperature accumulation profiles 400A to deliver effective thermal dosage to a target to effectively ablate tissue without damaging adjacent healthy tissue, energy output from ablation probe 130 may be varied over time, such as by heating probe 130, cooling probe 130, cycling on and off the supply of energy to probe 130, shutting down probe 130, or the like. Implementing one of the temperature accumulation profiles 400A may be facilitated using one or more temperature sensors TS, which continually monitor and provide temperature data, as detailed below. Computing device 100 may further incorporate an algorithm that uses the feedback from temperature sensors TS to repeatedly calculate and adjust, at intervals or continuously, the appropriate effect, e.g., the energy output, heating, cooling, on/off cycling, etc., required to implement and maintain the selected temperature accumulation profile 400A.

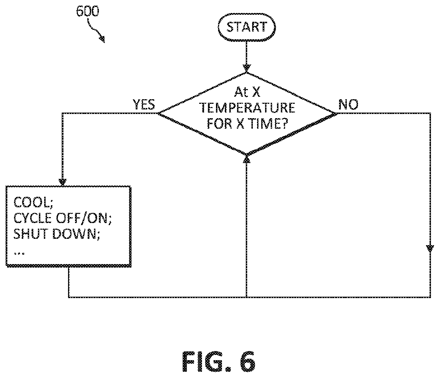

Referring now to Fig. 6, a safety protocol 600 may be implemented, together with or separate from temperature accumulation profiles 400A (FIG. 4), in accordance with temperature feedback as a function of time, to provide an integrated, rather than hard coded interlock. While temperature accumulation profiles 400A of treatment plan 400 (see FIG. 4) are used to automatically adjust energy output, heating/cooling, etc. of the probe 130 (FIG. 1) to provide an appropriate thermal dosage over the course of an ablation procedure based on a treatment plan 400 selected, safety protocol 600 is configured to monitor and dynamically adjust energy output if a potentially tissue-damaging condition is detected. Safety protocol 600 may be a unitary program(s) and/or a sub-program(s) that is part of application 216 (FIG. 2).

In embodiments, similarly as with temperature accumulation profiles 400A (FIG. 4A), safety protocol 600 may be implemented to: automatically increase or decrease energy output, such as by heating or cooling, rapidly or gradually; automatically shut off energy output to treatment system 10 and/or ablation probe 130 (see FIG. 1); and/or automatically cycle energy output on/off. Safety protocol 600 operates to prevent system 10 from operating at high temperatures for a period of time determined to potentially result in damage to healthy tissue. For example, hard-coded interlocks are unable to prevent potential damage to tissue in situations where a threshold temperature is set to 60 degrees Celsius and tissue is heated to 59 degrees Celsius for two minutes, despite the fact that such heating of tissue may have the same or worse effect as it would be to heat tissue to 60 degrees Celsius for one minute. An interlock integrated over time is capable of preventing the above-scenario by not only considering temperature but also considering the length of time tissue is at a particular temperature. Safety protocol 600 may include a plurality of "rules" regarding different temperatures and different durations of time. Further, various different safety protocols 600 may be provided with different sets of "rules," depending upon tissue type or other characteristics, similarly as detailed above with respect to temperature accumulation profiles 400A (FIG. 4A), thus enabling the user to select a particular safety protocol 600 prior to use.

Referring back to Fig. 1, temperature sensors TS may be utilized with treatment system 10 to constantly observe/monitor tissue temperatures in, or adjacent to, an ablation zone to enable implementation of temperature accumulation profiles 400A (FIG. 4A) and/or safety protocols 600 (FIG. 6). For example, one or more temperature sensors TS may be provided on the ablation probe 130, e.g., adjacent the distal radiating section, and may be configured to measure tissue temperatures in or adjacent to an ablation zone. It should be appreciated that temperature sensors TS may be placed at any suitable location in treatment system 10 to provide temperature information, such as within a generator (not shown), catheters (not shown), proximal and/or distal radiating sections of ablation probe 130, the patient's body, or the like. As such, temperature sensors TS may be configured to provide a greater array of temperature data collection points and provide greater detail on tissue temperature during and/or following an application of energy from ablation probe 130. In an embodiment, temperature sensors TS may also be used to monitor impedance, which may be useful in predicting coagulum formation. Impedance monitoring may be correlated with temperature monitoring for quantitative tissue injury predictions. The temperature sensors TS may be, for example, a radiometer or thermocouple based system, a magnetic resonance imaging (MRI) thermometry system, or any other tissue temperature monitoring system known in the art.

In embodiments, the temperature sensor TS may be a remote temperature probe(s) placed in at least one location of treatment system 10. The remote temperature probe may be a thermocouple or a thermistor and may be incorporated into or provide feedback to computing device 100 and/or a user (e.g., audio, visual, and/or tactile feedback) during the procedure. The temperature sensors TS may be configured to continuously output a temperature signal to computing device 100, display 110, and/or display 206, or may do so at pre-determined intervals. A real-time clock (not explicitly shown), timer, or other suitable time-measuring component associated with computing device 100 enables correlations of the temperatures received from temperature sensors TS to time so as to enable implementation of the above-detailed temperature accumulation profiles 400A (FIG. 4A) and/or safety protocols 600 (FIG. 6).

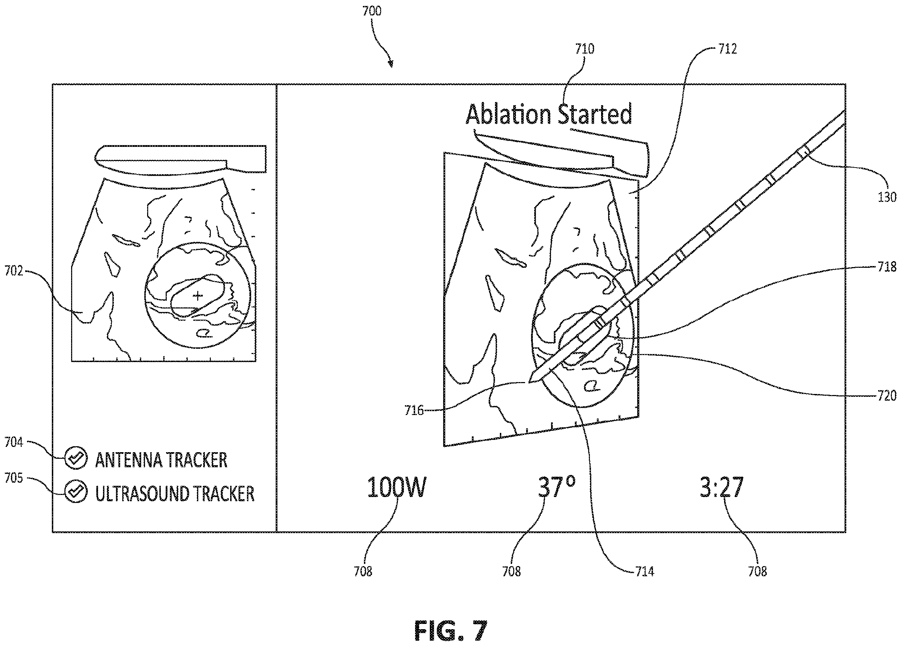

Referring now to Fig. 7, an example screen 700, which may be displayed on display 110 during a microwave ablation procedure, is shown. Screen 700 includes a view 702 of the live 2D ultrasound images captured during the procedure. Screen 700 further shows a status indicator 704 for ablation probe 130 and a status indicator 706 for ultrasound sensor 140. Screen 700 may also include a view 708 for displaying status messages relating to the ablation procedure such as the temperature accumulation profile 400A (FIG. 4) and/or safety protocols 600 (FIG. 6) being utilized, a power setting of ablation probe 130, duration of ablation at a specific temperature, elapsed time of the ablation and/or a time remaining until the ablation procedure is complete, progression of the ablation, feedback from a temperature sensor TS (FIG. 1), and a zone chart used during the ablation procedure. Screen 700 further includes a view 710 for showing transient messages relating to the ablation procedure. Screen 700 also displays the navigation view 712, in which ablation probe 130 as well as a shadow indicator 714 representing the portion of ablation probe 130 which lies below the ultrasound imaging plane, a vector line 716 representing the trajectory of the ablation probe 130, a current ablation zone 718 showing the area which is currently being ablated, and a total ablation zone 720 showing the area which will be ablated if the ablation procedure is allowed to run to completion, are shown.

In an embodiment, screen 700 may provide a user with ultrasonic EM, and/or infrared visualization of the target tissue, adjacent healthy tissue, such that thermal dosage and/or damage may be assessed to the tissue structures. In an embodiment, display screen 700 may provide audio and/or visual feedback alerting a user to an unsafe condition. For example, screen 700 may indicate to a user that current ablation zone 718 and/or total ablation zone 720 is moving outside the target area in close proximity to other tissue structures and that an unsafe condition is imminent. Display screen 700 may also provide audio and/or visual feedback to ensure that treatment plan 400 and temperature accumulation profiles 400A (FIG. 4) and/or safety protocols 600 (FIG. 6) are operating within established parameters.

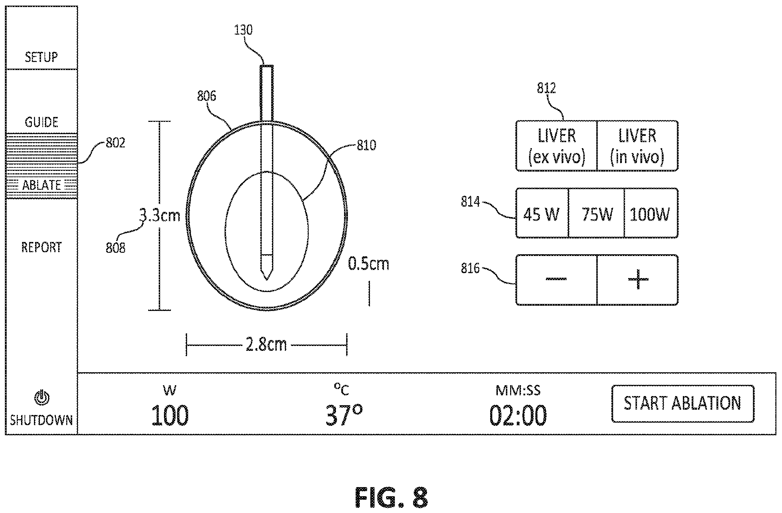

Referring now to Fig. 8, an example screen 800, which may be displayed on display 206 during the ablation step of the microwave ablation procedure, is shown. Screen 800 shows an indicator 802 that the system is now operating in the ablation step. Screen 800 further shows the surgical tool currently being used during the procedure, in the example of ablation probe 130, and the ablation zone 806 based on the configured power and size of ablation probe 130, as well as the dimensions 808 of the ablation zone and a distance from the distal end of ablation probe 130 to the edge of the ablation zone. Screen 800 may also include a view 809 for displaying status messages relating to the ablation procedure such as the temperature accumulation profile 400A (FIG. 4) and/or safety protocol 600 (FIG. 6) being utilized, a power setting of ablation probe 130, duration of ablation at a specific temperature, total duration of the ablation and/or a time remaining until the ablation procedure is complete, progression of the ablation, feedback from a temperature sensor TS (FIG. 1), and a zone chart used during the ablation procedure.

Screen 800 also shows a progress indicator 810 representing the progress of the ongoing ablation relative to ablation probe 130 and projected ablation zone 806. Screen 800 further includes a button 812 allowing the clinician to select a desired ablation zone chart based on the temperature accumulation profile 400A (FIG. 4), the anatomical location of ablation probe 130, and/or in vivo or ex vivo data. Screen 800 also includes a button 814 allowing the user to select a power setting for ablation probe 130, and a button 816 allowing the clinician to increase or decrease the size of the ablation zone based on the selected ablation zone chart.

Although embodiments have been described in detail with reference to the accompanying drawings for the purpose of illustration and description, it is to be understood that the inventive processes and apparatus are not to be construed as limited thereby. It will be apparent to those of ordinary skill in the art that various modifications to the foregoing embodiments may be made without departing from the scope of the disclosure.

## Claims

1. A microwave ablation system (10), comprising:
a microwave ablation probe (130) configured to deliver energy to a target volume of tissue during an ablation procedure;
at least one temperature sensor configured to determine a temperature of the target volume of tissue at a plurality of points in time during the ablation procedure; and
a computing device (100) operably coupled to the microwave ablation probe and the at least one temperature sensor, the computing device including a processor (204) and a memory (202) storing instructions, which when executed by the processor, cause the computing device to:
load a temperature accumulation profile (400A) corresponding to the target volume of tissue, the temperature accumulation profile providing a change in tissue temperature as a function of time that results in effective ablation of the target tissue, wherein the computing device is configured to load the temperature accumulation profile according to a user selection of (i) a category of target tissue and (ii) a sub-category comprising a list of temperature accumulation profiles all of which are applicable to the ablation of the selected target tissue; and
dynamically control at least one setting of the microwave ablation probe in accordance with the temperature of the target volume of tissue determined by the at least one temperature sensor at each of the points in time such that the temperature of the target volume of tissue follows the temperature accumulation profile during the ablation procedure.

2. The system of claim 1, wherein the at least one temperature sensor includes a plurality of temperature sensors.

3. The system of claim 2, wherein at least one of the plurality of temperature sensors is disposed on the microwave ablation probe.

4. The system of claim 2, wherein at least one of the plurality of temperature sensors is a remote temperature probe.

5. The system of any preceding claim, wherein the at least one setting of the microwave ablation probe includes at least one of: energy output from the microwave ablation probe, cooling of the microwave ablation probe, or cycling the microwave ablation probe on and off.

6. The system of any preceding claim, wherein the computing device includes a timer configured to correlate the temperature of the target volume of tissue at each of the points in time to an elapsed time of the ablation procedure.

7. The system of any preceding claim, wherein the computing device dynamically controls the at least one setting of the microwave ablation probe based upon the Arrhenius equation.

8. The system of any preceding claim, further comprising an ultrasound imager (140) configured to generate real-time ultrasound images.

## Patentansprüche

1. Mikrowellenablationssystem (10), das Folgendes umfasst:
eine Mikrowellenablationssonde (130), die konfiguriert ist, um während eines Ablationsverfahrens Energie an ein Zielvolumen von Gewebe abzugeben;
wenigstens einen Temperatursensor, der konfiguriert ist, um eine Temperatur des Zielvolumens von Gewebe zu mehreren Zeitpunkten während des Ablationsverfahrens zu bestimmen; und
eine Rechenvorrichtung (100), die mit der Mikrowellenablationssonde und dem wenigstens einen Temperatursensor wirkgekoppelt ist, wobei die Rechenvorrichtung einen Prozessor (204) und einen Speicher (202) einschließt, der Anweisungen speichert, die, wenn sie durch den Prozessor ausgeführt werden, die Rechenvorrichtung zu Folgendem veranlassen:
Laden eines Temperaturakkumulationsprofils (400A), das dem Zielvolumen von Gewebe entspricht, wobei das Temperaturakkumulationsprofil eine Änderung einer Gewebetemperatur in Abhängigkeit von Zeit bereitstellt, die in einer wirksamen Ablation des Zielgewebes resultiert, wobei die Rechenvorrichtung konfiguriert ist, um das Temperaturakkumulationsprofil gemäß einer Benutzerauswahl von (i) einer Kategorie von Zielgewebe und (ii) einer Unterkategorie, die eine Liste von Temperaturakkumulationsprofilen umfasst, die alle auf die Ablation des ausgewählten Zielgewebes anwendbar sind, zu laden; und
dynamisches Steuern wenigstens einer Einstellung der Mikrowellenablationssonde gemäß der Temperatur des Zielvolumens von Gewebe, die durch den wenigstens einen Temperatursensor zu jedem der Zeitpunkte derart bestimmt wird, dass die Temperatur des Zielevolumens von Gewebe dem Temperaturakkumulationsprofil während des Ablationsverfahrens folgt.

2. System nach Anspruch 1, wobei der wenigstens eine Temperatursensor mehrere Temperatursensoren einschließt.

3. System nach Anspruch 2, wobei wenigstens einer der mehreren Temperatursensoren an der Mikrowellenablationssonde angeordnet ist.

4. System nach Anspruch 2, wobei wenigstens einer der mehreren Temperatursensoren eine entfernte Temperatursonde ist.

5. System nach einem der vorhergehenden Ansprüche, wobei die wenigstens eine Einstellung der Mikrowellenablationssonde Folgendes umfasst: Energieabgabe von der Mikrowellenablationssonde, Kühlung der Mikrowellenablationssonde und/oder Ein- und Ausschalten der Mikrowellenablationssonde.

6. System nach einem der vorhergehenden Ansprüche, wobei die Rechenvorrichtung einen Zeitgeber einschließt, der konfiguriert ist, um die Temperatur des Zielvolumens von Gewebe zu jedem der Zeitpunkte mit einer verstrichenen Zeit des Ablationsverfahrens zu korrelieren.

7. System nach einem der vorhergehenden Ansprüche, wobei die Rechenvorrichtung die wenigstens eine Einstellung der Mikrowellenablationssonde basierend auf der Arrhenius-Gleichung dynamisch steuert.

8. System nach einem der vorhergehenden Ansprüche, das ferner einen Ultraschallbildgeber (140) umfasst, der konfiguriert ist, um Echtzeit-Ultraschallbilder zu erzeugen.

## Revendications

1. Système d'ablation par micro-ondes (10) comprenant :
une sonde d'ablation par micro-ondes (130) conçue pour alimenter en énergie un volume cible de tissu pendant une procédure d'ablation ;
au moins un capteur de température conçu pour déterminer une température du volume cible de tissu à une pluralité de points dans le temps pendant la procédure d'ablation ; et
un dispositif informatique (100) couplé de manière fonctionnelle à la sonde d'ablation par micro-ondes et à l'au moins un capteur de température, le dispositif informatique comportant un processeur (204) et une mémoire (202) stockant des instructions qui, lorsqu'elles sont exécutées par le processeur, amènent le dispositif informatique à :
charger un profil d'accumulation de température (400A) correspondant au volume cible de tissu, le profil d'accumulation de température fournissant un changement de température tissulaire en fonction du temps qui entraîne une ablation effective du tissu cible, le dispositif informatique étant configuré pour charger le profil d'accumulation de température selon une sélection utilisateur (i) d'une catégorie de tissu cible et (ii) d'une sous-catégorie comprenant une liste de profils d'accumulation de température qui sont tous applicables à l'ablation du tissu cible sélectionné ; et
commander dynamiquement au moins un réglage de la sonde d'ablation par micro-ondes selon la température du volume cible de tissu déterminée par l'au moins un capteur de température à chacun des points dans le temps de telle sorte que la température du volume cible de tissu suit le profil d'accumulation de température pendant la procédure d'ablation.

2. Système selon la revendication 1, dans lequel l'au moins un capteur de température comporte une pluralité de capteurs de température.

3. Système selon la revendication 2, dans lequel au moins l'un de la pluralité de capteurs de température est disposé sur la sonde d'ablation par micro-ondes.

4. Système selon la revendication 2, dans lequel au moins l'un de la pluralité de capteurs de température est une sonde de température à distance.

5. Système selon l'une quelconque des revendications précédentes, dans lequel l'au moins un réglage de la sonde d'ablation par micro-ondes comporte : une sortie d'énergie de la sonde d'ablation par micro-ondes, et/ou un refroidissement de la sonde d'ablation par micro-ondes, et/ou un cycle de mise en marche et d'arrêt de la sonde d'ablation par micro-ondes.

6. Système selon l'une quelconque des revendications précédentes, dans lequel le dispositif informatique comporte une minuterie configurée pour corréler la température du volume cible de tissu à chacun des points dans le temps à un temps écoulé de la procédure d'ablation.

7. Système selon l'une quelconque des revendications précédentes, dans lequel le dispositif informatique commande dynamiquement l'au moins un réglage de la sonde d'ablation par micro-ondes sur la base de l'équation d'Arrhénius.

8. Système selon l'une quelconque des revendications précédentes, comprenant en outre un imageur échographique (140) configuré pour générer des images d'échographie en temps réel.
